# EUROPÄISCHE PATENTSCHRIFT

(11) **EP 1 624 051 B1**
(45) Veröffentlichungstag und Bekanntmachung des Hinweises auf die Patenterteilung: **10.06.2015**
(21) Anmeldenummer: 05016689.1
(22) Anmeldetag: 01.08.2005
(51) Int. Cl.: C12M 1/107, C12M 1/36, C12P 5/02

(54) **Verfahren zur Vergärung von Biomasse**
Process for the fermentation of biomass
Procédé de fermentation de biomasse

(30) Priorität: 03.08.2004 DE 102004037798
(43) Veröffentlichungstag der Anmeldung: 08.02.2006
(73) Patentinhaber: Hochschule für angewandte Wissenschaften Hamburg, 20099 Hamburg (DE)
(72) Erfinder: Scherer, Paul, 21029 Hamburg (DE)
(74) Vertreter: Stüven, Ralf

(56) Entgegenhaltungen:
- DD-A1- 277 701
- DE-A1- 10 316 680
- DE-A1- 19 518 983
- US-A- 5 470 745
- PATZWAHL, S. ET AL.: "Microcontroller-Based Fuzzy System to Optimize the Anaerobic Digestion in Biogas Reactors" 2001, SPRINGER-VERLAG , BERLIN(GERMANY) , XP002371057 IN: REUSCH, B (ED.): Computational Intelligence, Theory and Applications, International Conference, 7th Fuzzy Days, Dortmund, Germany, October 1-3, 2001, Proceedings. * Seite 2 - Seite 10 *

## Beschreibung

Die Erfindung betrifft ein Verfahren zur Vergärung von Biomasse, insbesondere von pflanzlichen nachwachsenden Rohstoffen und Speiseresten.

### Hintergrund der Erfindung

Verfahren zur Vergärung von festen organischen Reststoffen, beispielsweise aus der Landwirtschaft und der Lebensmittelindustrie, sind seit längerem bekannt (s. z.B. weiland, P., Industrieabfälle und Möglichkeiten für deren Vergärung, In: Pfirter, A. (Hrsg.), Vergärung fester organischer Abfälle - Möglichkeiten und Grenzen der Biogasgewinnung, Schriftenreihe des Arbeitskreises für die Nutzbarmachung von Siedlungsabfällen e.V. (ANS), Heft 16, S. 54-83, 1989, Basel). Die in der Biomasse enthaltenen polymeren organischen Moleküle werden mit Hilfe von Mikroorganismen unter Sauerstoffabschluß (d.h. anaerob) abgebaut, wobei u.a. Methan gebildet werden kann, so daß auch von einer Biomethanisierung gesprochen wird.

Hinsichtlich der Verfahrensführung kann z.B. zwischen ein- und zweistufen Verfahren unterschieden werden. Bei einstufigen Verfahren werden die Abbauschritte (1) Hydrolyse der organischen Polymere, (2) Versäuerung der Hydrolyseprodukte (A-cidogenese), (3) Acetatbildung (Acetogenese) und (4) Methanbildung (Methanogenese) in einem einzigen Reaktorsystem durchgeführt, während bei zweistufen verfahren Hydrolyse und versäuerung apparativ von der Acetogenese und Methanogenese getrennt ablaufen. Das erhaltene Gasgemisch, das neben Methan und CO₂ beispielsweise noch in Spuren H₂S, Stickstoff (N₂), Wasserstoff (H₂) und Kohlenmonoxid (CO) enthält oder enthalten kann, wird häufig als Biogas bezeichnet.

Auch die Gärtemperatur hat Einfluß auf den Gärprozeß. In Bezug auf die Gärtemperatur wird zwischen mesophiler und thermophiler Betriebsweise unterschieden. Im Temperaturbereich von etwa 25-40 °C spricht man in der Regel von mesophiler Betriebsweise, bei Temperaturen im Bereich von etwa 41-60 °C von thermophiler Betriebsweise. Ab 65 °C kann man auch von hyperthermophiler Vergärung sprechen (P. Scherer, G. Vollmer, T. Fakhouri, S. Martensen: Development of a methanogenic process to degrade exhaustively organic fraction municipal "grey waste" under thermophilic and hyperthermophilic conditions. Water Science & Technology Vol.41 (Nr. 3), S. 83-91,2000).

Weiterhin kann nach dem eingesetzten zu vergärenden Substrat zwischen Monovergàrung und Kovergärung unterschieden werden, wobei bei der Monovergärung weit überwiegend ein einziges Substrat (beispielsweise Gülle) eingesetzt wird, während bei der Kovergärung beispielsweise zusätzlich zu tierischen Ausscheidungen organische Abfallstoffe, z.B. Fette und Speiseabfälle, aus Nahrungsmittelindustrie, Gewerbe und kommunaler Entsorgung mit vergoren werden. Solche zusätzlich zu einem Hauptsubstrat vergorenen Substrate werden als Kosubstrate bezeichnet.

Einstufige Verfahren werden in der Praxis den zweistufigen häufig aus Kostengründen bevorzugt. Nachteil insbesondere der bekannten einstufigen Verfahrensweise ist, daß es häufig nicht gelingt, einen stabilen und effizienten Betrieb zu erhalten. Besonders im Falle der Monovergärung relativ leicht fermentierbarer Biomasse kann es dazu kommen, daß die vergleichsweise empfindliche Methanogenese gestört wird oder gar zum Erliegen kommt. Beispielsweise kann es bei stärke- und kohlenhydxathaltigen Biomassen aufgrund des hohen C/N-Verhältnisses (Verhältnis von Kohlenstoff zu Stickstoff in den zu vergärenden Substraten) zu starker und rauscher Säurebildung kommen, die die Methanbildung hemmt. Im Falle von stark eiweiß- beziehungsweise stickstoffhaltigen Substraten (z.B. Speisereste, Schlempen) kann es zu einer überschießenden Ammoniak- und schwefelwasserstoffbildung kommen, was ebenfalls zu einer Hemmung der Methanbildung führen kann. Wenn es zu einem Zusammenbruch der Methanbildung gekommen ist, dauert es häufig sehr lange (oft Monate), bis der Prozeß seine Ausgangsleistung wiedererlangt. Es ist versucht worden, diesem Problem durch Kovergärung (z.B. mit Gülle) öder durch eine zweistufige Verfahrensweise zu begegnen. In der DE 19516378 ist beispielsweise ein zweistufiges thermophiles Vergärungsverfahren beschrieben. Zweistufige Anlagen weisen aber u.a. den Nachteil auf, daß sie vergleichsweise aufwendig und damit teuer sind.

Liu, J., Olsson, G., Mattiasson, B. (2004), Monitoring and Control of an Anaerobic Upflow Fixed-Bed Reactor for High-Loading-Rate Operation and Rejection of Disturbances, Biotechnol. Bioeng. 87, S. 43-53, beschreiben ein Aufstrom-Festbettverfahren zum anaeroben Abbau von Biomasse, bei dem der pH, die Gasflussrate und der Methangehalt online gemessen werden. Als realistischste Prozessvariablen zur Überwachung des Prozesses werden der pH, die Biogasbildungsrate und die Differenz zwischen der tatsächlichen Biogasflussrate und deren Sollwert genannt.

Bislang ist es nicht gelungen, ein Vergärungsverfahren für Biomasse, insbesondere für leicht vergärbare, sogenannte nachwachsende Rohstoffe, d. h. pflanzliche organische Materialien, und hocheiweißhaltige Speisereste oder Schlempen, bereitzustellen, das einfach zu handhaben ist und eine zufriedenstellende Betriebsstabilität aufweist. Bekannte Verfahren sind darüber hinaus häufig ineffizient, da lange Aufenthaltszeiten und somit auch große Reaktorvolumina erforderlich sind und/oder kein weitgehend vollständiger Abbau des Substrats erfolgt. Darüber hinaus sind die erzielten Gasbildungsraten vergleichsweise gering. Die durchschnittliche Verweildauer des zu vergärenden Substrats kann in bestehenden Vergärungsanlagen beispielsweise 30 Tage und länger betragen, wobei ohne Zuführung von Fetten lediglich volumetrische Gasbildungsraten von deutlich unter 4 m³/(m³ · d) erreicht werden. (G. Langhans: Bemessung und Bilanzierung der Biogasausbeuten in der Abfallvergärung. Abfallwirtschaftsjournal 1- 2, S. 35/38, 1997).

Aufgabe der vorliegenden Erfindung ist es, ein Vergärungsverfahren für Biomasse, insbesondere für sogenannte nachwachsende Rohstoffe, d. h. pflanzliche organische Materialien, sowie hocheiweißhaltige Substrate und Speisereste, bereiitzustellen, das die Nachteile des Standes der Technik nicht aufweist, insbesondere leicht durchzuführen, betriebsstabil und effizient ist, d.h. insbesondere hohe Raumzeitausbeuten ermöglicht.

Die Lösung der obigen Aufgaben erfolgt durch den Gegenstand des Anspruchs 1.

Bei dem erfindungsgemäßen Verfahren ist eine multivariable Regelung vorgesehen, d.h. eine Regelung, bei der mehr als ein Parameter in die Regelung eingeht, wobei als Eingangsvariablen (a) der pH-Wert, und (b) die Methankonzentration im gebildeten Biogas und (c) die spezifische Biogasbildungsrate eingesetzt werden. Alle diese Parameter sind leicht und zuverlässig mittels entsprechender Meßsonden beziehungsweise Meßgeräte, die auf dem Markt verfügbar und vergleichsweise kostengünstig erhältlich sind, online meßbar. Die Bestimmung weiterer Parameter, wie beispielsweise der Alkalinität, der Wasserstoffkonzentration, der Kohlendioxidkonzentration, der Kohlenmonoxidkonzentration und der Konzentration gelöster Fettsäuren (volatile fatty acids, VFA) ist nicht erforderlich, so daß sich der Einsatz weiterer, u. U. teuerer und nicht über längere Zeit stabil betreibbarer, Meßgeräte erübrigt.

Die Erfassung von Offline-Daten ist ebenfalls nicht erforderlich, so daß der ganze Prozeß vollautomatisch ablaufen kann. Einzig die pro Zeiteinheit zugeführte organische Masse muß in etwa bekannt sein. Hierzu kann es aber ausreichen, die organische Masse einmalig offline zu bestimmen. Auch eine Schätzung dieses Wertes kann ausreichen, obwohl es natürlich bevorzugt ist, wenigstens einmal eine genaue Messung vorzunehmen und diese gegebenenfalls von Zeit zu Zeit, am besten regelmäßig, zu überprüfen. Auf diese Weise ist ein kostengünstiger, stabiler und einfacher Betrieb einer Vergärtungsanlage möglich, wobei hohe Biogasbildungsraten sowie hohe Substratdurchsätze erreicht werden können. So sind volumetrische Gasbildungsraten von 4 m³ pro m³ Substrat und Tag (d) und mehr (z.B. 6 m³ pro m³) bei Verweildauern von weit unter 30 Tagen, beispielsweise 7 Tagen, möglich.

Der pH-Wert kann leicht, zuverlässig und auch über längere Zeit stabil online gemessen werden. Entsprechende Meßsonden beziehungsweise Meßgeräte sind auf dem Markt verfügbar und vergleichsweise kostengünstig erhältlich. Die Messung der Methankonzentration im Biogas neben der Biogasbildungsrate ist vorteilhaft, um feststellen zu können, ob eine gemessene Biogasbildung tatsächlich im wesentlichen auf die gewünschten Gärungsprozesse durch Methanbildner zurückgeht oder ob beispielsweise Hefen zugegen sind, die lediglich CO₂, jedoch kein Methan bilden, wodurch eine Biogasbildung vorgetäuscht würde. Die Gasbildungsrate wird unter Umständen durch Hefen kaum beeinflußt, die Gesamtleistung des Prozesses verschlechtert sich jedoch deutlich. Die Messung der Gasbildungsrate ist vorteilhaft, um die aktuelle Leistung des Systems beurteilen zu können und ist insbesondere in Anfahrphasen sowie in Erholungsphasen eine wertvolle Meßgröße.

Unter dem hier verwendeten Begriff "Verweildauer" wird die Zeit verstanden, die sich das Substrat durchschnittlich in dem Behälter (Reaktor), in dem der Vergärungsprozeß stattfindet, aufhält. Unter "Biogasbildungsrate" wird die pro Zeiteinheit gebildete Menge Biogas verstanden. Die "volumetrische Biogasbildungsrate" ist die pro Zeiteinheit und Volumen vergorenen Substrats gebildete Menge Biogas. Bei der "spezifischen Biogasbildungsrate" wird die Biogasbildungsrate nicht auf das Volumen des durchgesetzten Substrats bezogen, sondern auf den Gehalt an organischem Material in dem Substrat, der beispielsweise als Masse organischer Trockensubstanz (oTS) angegeben wird. Die spezifische Biogasbildungsrate (spez. GPR) ist eine verrechnete Größe zur Gasbildung. Sie wird errechnet aus der Gasbildung je Volumeneinheit Substrat und Zeiteinheit, wobei der organische Trockensubstanzgehalt pro Volumeneinheit Substrat anstelle der Volumeneinheit eingesetzt wird. Die spezifische Biogasbildungsrate sagt direkt etwas über die Abbaurate aus und wird beispielsweise in Liter gebildetem Biogas pro Gramm oTS des vergorenen Substrats angegeben.

Vorzugsweise ist die Regelung eine Fuzzy-Regelung. Die Fuzzy-Regelungstechnik ist eine Regeltechnik, bei der versucht wird, ein festgelegtes Regelziel über ein auf Erfahrungswerten beruhendes System von "unscharf" formulierten Steuerregeln zu erreichen. Das besondere Kennzeichen von Fuzzy-Reglern sind linguistische Variable, die. einen Satz von Regeln ergeben. Bei den linguistische Variablen handelt es sich um sprachliche Terme, die unscharfe Ausdrücke wie zum Beispiel "niedrig", "zu hoch" oder "gut" enthalten. Der scharfe Zahlenwert einer Eingangsvariablen wird in einem Fuzzy-Regelungssystem durch die linguistischen Variablen, mithin als durch einen unscharfen sprachlichen Ausdruck, repräsentiert. Dabei werden in einem ersten Schritt ("Fuzzifizierung") exakte Meßwerte in linguistische Ausdrücke überführt. In einem nächsten Schritt ("Inferenz") wird dann anhand der Fuzzy-Regeln ein Ergebnis in Form von linguistischen Ausgangsvariablen ermittelt. In einem dritten Schritt ("Defuzzifizierung") werden die linguistischen Ausgangsvariablen in exakte Ausgangswerte zurückverwandelt.

Es hat sich herausgestellt, daß eine Fuzzy-Regelungstechnik, bei der die drei Eingangsvariablen (a) pH-Wert des Substrats im Reaktor, (b) spezifische Biogasbildungsrate und (c) Methankonzentration im Biogas die einzigen Eingangsvariablen sind, eine besonders zuverlässige, kostengünstige und zugleich effiziente Vergärung von Biomasse ermöglicht. Um eine möglichst einfache Regelung zu erhalten, ist es vorteilhaft, die Zahl der Fuzzy-Regeln so klein wie möglich zu halten. Bei dem vorliegenden Verfahren werden in einer bevorzugten Ausführungsform lediglich 27 Fuzzy-Regeln verwendet. Die Ausgestaltung der einzelnen Fuzzy-Regeln hängt im Detail vom zugegebenen Substrat ab.

Mit Hilfe des erfindungsgemäßen Verfahrens werden zum einen vorzugsweise konzentrierte Biomassen pflanzlicher Herkunft, insbesondere Abfälle aus der Landwirtschaft, beispielsweise Silage, behandelt. Zum anderen sind auch hocheiweißhaltige Abfälle aus der Nahrungsmittelindustrie, beispielsweise Speisereste und Schlempen, ein bevorzugtes Substrat für das erfindungsgemäße Verfahren. Es handelt sich dabei vorzugsweise um eine sogenannte Monovergärung von Feststoffen, bei der im wesentlichen Substrate einheitlicher Zusammensetzung bzw. Herkunft, und keine zusätzlichen Substrate aus anderen Quellen, wie beispielsweise Gülle, verwendet werden. Weiter bevorzugt handelt es sich um ein Naßvergärungsverfahren. Unter Naßvergärung soll hier eine Vergärung bei Trockensubstanzgehalten (TS) des Substrates von bis zu 15 % verstanden werden. Unter Trockenvergärungsverfahren werden hier Verfahren verstanden, bei denen die Substrate höhere TS-Gehalte aufweisen, z.B. 25-35 %.

Das verfahren wird bevorzugt bei Temperaturen von 4 bis 70 °C betrieben. Insbesondere bei pflanzlichen Biomassen wird das vorliegende Verfahren bevorzugt mesophil betrieben, d. h. in einem Temperaturbereich von 25-40 °C, besonders bevorzugt bei etwa 37 °C. Dies ist vorteilhaft, um den mit thermophilen Verfahren verbundenen vergleichsweise höheren apparativen und energetischen Aufwand zu vermeiden. Das Verfahren ist aber auch für eine thermophile Verfahrensweise geeignet, wobei die Temperatur bevorzugt >40-65 °, weiter bevorzugt >40-50 °C und besonders bevorzugt 42 -45 C beträgt.

Mit dem erfindungsgemäßen Verfahren können insbesondere bei festen Monosubstraten durchschnittliche Verweildauern des Substrats erreicht werden, die deutlich unter 30 Tagen liegen. Bevorzugt liegen die durchschnittlichen Verweildauern des Substrats bei 10 Tagen und darunter, weiter bevorzugt ≤ 8 Tagen und besonders bevorzugt ≤ 6 Tagen. Bei gelösten Substraten und Reaktoren mit Entkopplung der Feststoffverweilzeit (Aufenthaltszeit der Bakterienbiomasse) von der Flüssigverweilzeit (Aufenthalt der Flüssigkeit im Reaktor) sind noch viel kürzere Reaktoraufenthaltszeiten von unter 1 Tag möglich. Bei diesen geringen verweildauern sind kleine Behältervolumina erreichbar, was eine platzsparende und kostengünstige Bauweise ermöglicht.

Das erfindungsgemäße Verfahren wird vorzugsweise bei einem pH-Wert im neutralen Bereich, d. h. im Bereich zwischen pH 6 und pH 8, durchgeführt. Besonders bevorzugt beträgt der pH-Wert etwa 6,8-7,1.

Mit Hilfe des vorliegenden Verfahrens ist ein hoher Wirkungsgrad von Vergärungsanlagen erhältlich. Das zu vergärende Substrat kann dem Reaktor somit in hoher Rate zugeführt werden. Bevorzugt wird das Substrat dem Reaktor mit einer Rate von ≥ 5 g oTS • 1⁻¹ • d⁻¹, besonders bevorzugt von ≥ 10 g oTS • 1⁻¹ • d⁻¹ zugeführt.

In einer besonders bevorzugten Ausführungsform des erfindungsgemäßen Verfahrens wird Silage vergoren, d.h. zerkleinerte Biomasse (z.B. Mais, Gras, Klee, Luzerne, Ackerbohnen, Hafer, Rübenblätter usw), die bereits einer Vorvergärung (Milchsäuregärung) unterzogen wurde.

Im folgenden wird die Erfindung anhand von Ausführungsbeispielen näher beschrieben. Es zeigt:

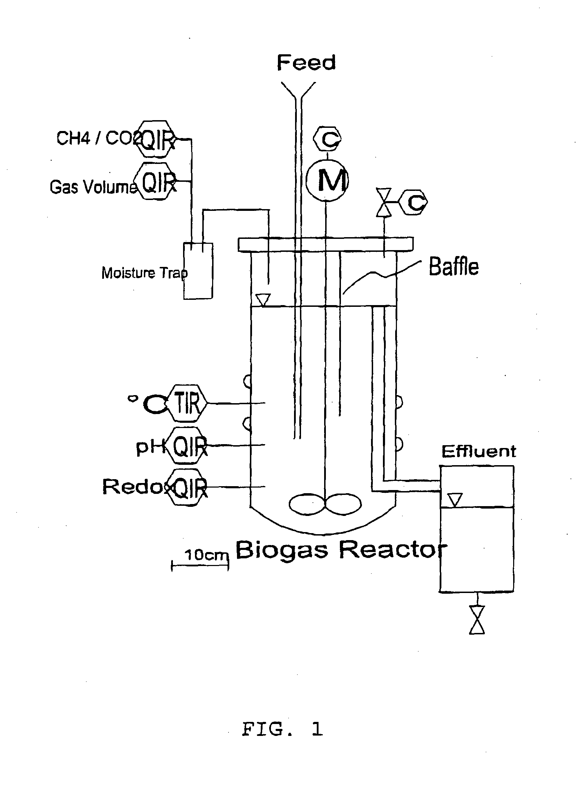

Figur 1 eine schematische Skizze einer im Labormaßstab eingesetzten Vergärungsanlage. Der Labor-Gärverauchsreaktor (5,7 Liter Reaktorfüssigkeit) besitzt eine Zwischenwand (Baffle). "Gas Volume (QIR)", Gasmengenmessung durch den MilligasCounter®; "CH4/CO2 QIR", Methan-/CO₂-Messung; "°C TIR", Temperaturmessung; "pH QIR", pH-Messung; "Redox QIR", Redoxpotentialmessung; M = Motor; c = controlled bzw. kontrolliert; Feed = Substratzugabe; Effluent = Auslauf; Moisture Trap = Kondenswasserfalle.

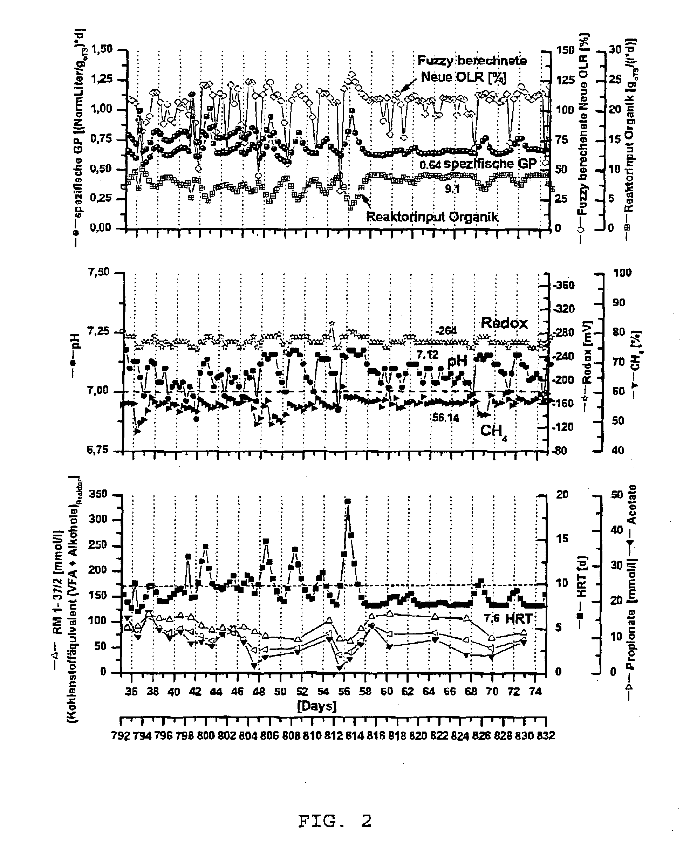

Figur 2 Ergebnisse eines Versuchs (Reaktortage 792-832) mit der in Figur 1 dargestellten Vergärungsanlage unter mesophilen Bedingungen. Dargestellt sind Werte, die für den Bioreaktor RM 1-37/2 erhalten wurden. GP = Biogasbildungsrate (gas production), OLR = Beladungsrate mit organischem Material (organic loading rate), oTS = organische Trockensubstanz, VFA = gelöste Fettsäuren (volatile fatty acids), HRT = Hydraulische Verweildauer (hydraulic retention time), Redox = Redoxpotential

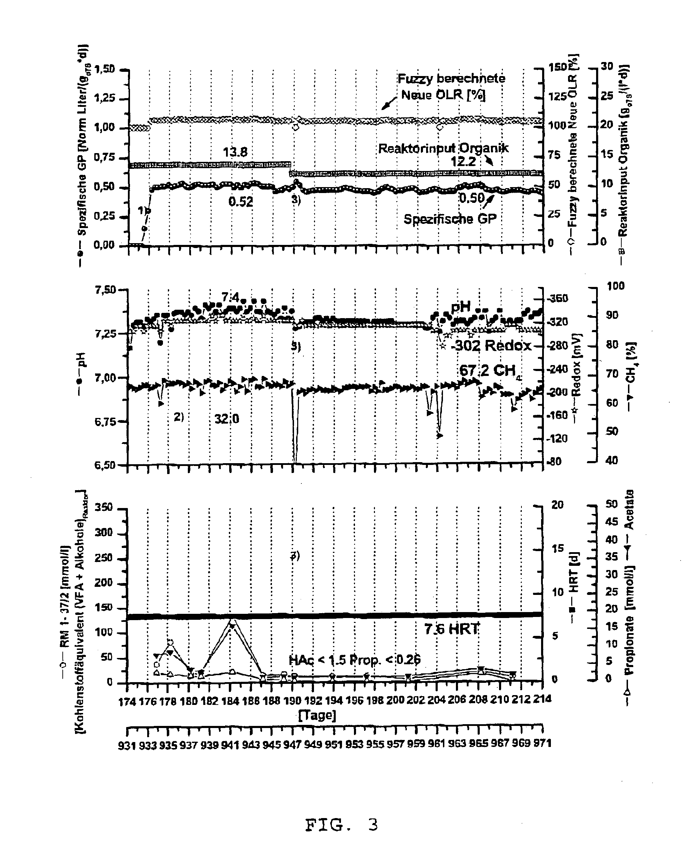

Figur 3 zeigt Ergebnisse des Versuchs aus Figur 2 zu einem späteren Zeitpunkt (Reaktortage 931-971) unter thermophilen Bedingungen. Die Temperatur betrug 42 °C. Die Bedeutung der Abkürzungen ist wie in Figur 2.

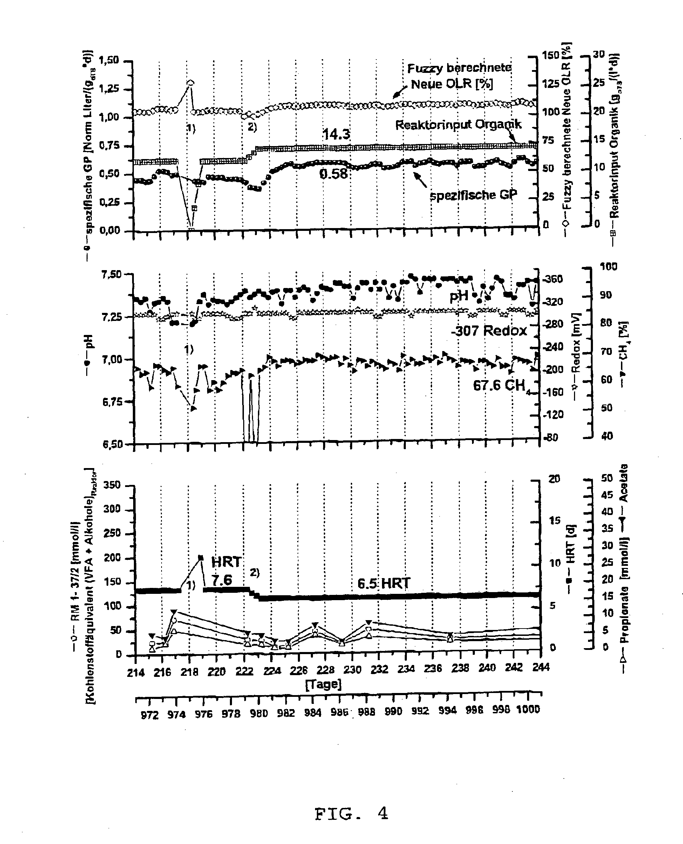

Figur 4 Ergebnisse der Fortsetzung (Reaktortage 971-1001) des Versuchs aus Figur 3. Die Bedeutung der Abkürzungen ist wie in Figur 2.

### 1. Mesophile Vergärung

Es wurden Versuche mit einer Vergärungsanlage im Labormaßstab durchgeführt. Die Anlage umfaßt drei parallel und unabhängig voneinander betreibbare Bioreaktoren, die jeweils ein Fassungsvolumen von 7 Litern mit einem Nutzvolumen von 5,7 Litern aufwiesen. Ein gekühltes Vorratsgefäß mit Rührer diente als Vorlage für das Substrat. Mit Hilfe einer Pumpe wurde Substrat aus dem vorratsgefäß in ein Dosiergefäß gefördert. Das Dosiergefäß war an einer elektronischen Waage aufgehängt, wodurch eine exakte Dosierung des Substrats ermöglicht wurde. An dem Dosiergefäß war ein pneumatisch betriebenes SchlauchQuetschventil angebracht. Unterhalb des Schlauch-Quetschventils befanden sich drei Trichter, die jeweils über einen Schlauch mit den drei Bioreaktoren verbunden waren und mit einem Schrittmotor positioniert wurden. Feststoffe aus dem Dosiergefäß rutschten bei Bestätigung des Schlauch-Quetschventils in die Reaktoren. Eine quantitative Zuführung von Feststoffen in die Reaktoren wurde durch die elektronische Waage sichergestellt. Über diese eine Waage konnten die 3 Reaktoren gleichzeitig sehr genau mit Substrat versorgt werden.

Die Anlage war mit einer Regelungseinheit versehen, bei der eine Fuzzy-Regelung implementiert war. Es wurden die drei Parameter pH-Wert im Reaktor, Methangehalt im Biogas und spezifische Biogasbildungsrate (als oTS • 1⁻¹ • d⁻¹) als Eingangsgrößen für die Regelung verwendet.

Ausgangsvariable war der Reaktorinput Organik bzw. die OLR (organic loading rate), also die Substratzuführ- bzw. -beladungsrate.

Die spezifische Biogasbildungsrate kann leicht aus der volumetrischen Biogasbildungsrate ermittelt werden, indem man den organischen Trockensubstanzgehalt eines bekannten Volumens (z.B. von 1 1) des Feststoffs nach bekannten verfahren ermittelt. Der organische Trockensubstanzgehalt ist bei Monosubatraten, wie z.B. Silage, Essensresten etc., im wesentlichen konstant, so daß es ausreicht, den entsprechenden Wert einmal, gegebenenfalls von Charge zu Charge erneut, zu bestimmen. Es kann auch ausreichen, mit einem Schätzwert zu arbeiten.

Als Substrat wurde Rübensilage (Rübe plus Blatt) mit einem mittleren pH-Wert von etwa 3,4 eingesetzt, ohne Zusätze. Für die Laborversuche wurde der Silagebrei zuvor mit Wasser von 14 g oTS/l auf 7,0 g oTS/l verdünnt. Ein Einsatz ohne vorherige Verdünnung ist aber ebenfalls möglich. Die Zufuhr der Silage erfolgte dreimal täglich im Abstand von 8 Stunden. Andere Beladungsintervalle können aber auch eingesetzt werden. Eine intervallartige Beladung sichert die für einen Hochlastbetrieb erforderliche stabile Mikroflora, weil sie diese besser "trainiert".

Vor jeder einzelnen Dosage wurde die zugeführte Substratmenge durch die Fuzzy-Regelung automatisch neu berechnet bzw. angepaßt. Die Minimal- und Maximalbeträge für die Dosage wurden vor dem Versuch festgelegt und richteten sich nach dem Stadium des Versuchs (Anfahrphase, Erfahrungsphase, Hochlastphase etc.) Die Versuchstemperatur lag bei 37 °C.

Die Figur 2 zeigt Ergebnisse eines Versuchs, bei dem nach längerem Anlagenbetrieb (am Tag 757) mit Rübensilage die beschriebene Fuzzy-Regelung gestartet wurde. Die Tage, an denen die Reaktoren mit Fuzzy-Regelung betrieben wurden, werden als "Fuzzy-Lauftage" bezeichnet. Dargestellt sind die "Fuzzy-Lauftage" 35-75 (bezogen auf die Gesamtlaufdauer des Experiments Tage 792 bis 832). Die drei Parallelreaktoren wurden kontinuierlich ohne Unterbrechung, d.h. auch nachts und an den Wochenenden, vollautomatisch betrieben. Das in den Figuren 2 und 3 dargestellte Redoxpotential ging nicht in die Fuzzy-Regelung ein, ebenso wie die Meßwerte für Propionsäure, Acetat und die Summe des gelösten Kohlenstoff. Die OLR ist in Prozent angegeben und bezieht sich auf den letzten Wert des letzten Zugabezyklus.

Aus Figur 2 ist ersichtlich, daß die Propionsäurekonzentration auf nahezu 20 mM (1.500 mg/l) anstieg, was im Falle saurer Silage als kritisch zu bezeichnen ist. Dennoch konnte mit Hilfe des erfindungsgemäßen Verfahrens über einen längeren Zeitraum ein hoher Substratumsatz und eine hohe Biogas-Ausbeute bei gleichzeitig stabiler vollautomatischer Betriebsweise aufrechterhalten werden. Ein Zusammenbruch des vergärungsprozesses konnte wirksam verhindert werden. Aufgrund der implementierten Fuzzy-Regelung, die lediglich die drei Variablen pH-Wert im Reaktor, Methangehalt im Biogas und spezifische Biogasbildungsrate in die Fuzzy-Regelung einbezog, konnte eine zu starke Ansäuerung und damit eine Beeinträchtigung der Mikroorganismen durch entsprechende Anpassung der Beladungsrate (OLR, Reaktorinput Organik) vermieden werden.

Die Beladungsrate betrug bei einer sehr kurzen verweildauer von <8 d etwa 8,8g oTS **•** l⁻¹ **•** d⁻¹ im Rührreaktor (126 ml • l⁻¹ **•** d⁻¹ multipliziert mit dem oTS-Gehalt von 7,0 g oTS/1). Die volumetrische Gasproduktion lag im Mittel bei 6,0• l⁻¹ • d⁻¹. Bei jeder Substratzu- und abgabe wurde der Reaktor vollständig durch Rühren bei 250 Upm für 5 min durchmischt. Dadurch gab es keine spezielle Imobilisierungsmöglichkeit für die Mikroflora, was eigentlich eher ungünstig ist, aber eine saubere Bilanzierung ermöglichte. Darüber hinaus können dadurch einfach gebaute Reaktoren ohne Entkopplung der Feststoff- von der Flüssigverweildauer eingesetzt werden.

Die eingesetzte Fuzzy-Regelung hat sich als geeignet für die mesophile Vergärung des vergleichsweise sauren Substrats Rübensilage erwiesen. In einer alternativen Ausführungsform kann sie noch dahingehend abgeändert werden, daß z.B. drei verschieden weite Regelfenster in der Fuzzy-Logik eingerichtet werden, die dreierlei verschieden genaue und dadurch verschieden schnelle Nachregelungen vorsehen. So kann beim Einfahren des Reaktors ein langsames bzw. vorsichtiges Erhöhen der Substratzufuhr, in der Stabilisierungsphase eine mittlere und bei Vollast eine schnelle Nachführung von Substrat gewählt werden. Die vorgefertigten Regelfenster können einen Zuammenbruch des Reaktors ebenfalls sicher verhindern, erleichtern aber in Fällen schlecht kalibrierter pH-Sonden oder eines plötzlichen Wechsels der pH-Elektrode durch pH-Verschiebung in Richtung des sauren Bereiches, was der Regelung irrtümlich eine Gefahr signalisieren würde, zusätzlich die Rückstellung in Richtung Vollastphase. Die Regelfenster können beispielsweise bei Bedarf einfach gewechselt werden.

Im Mittel wurden Biogasbildungsraten von 0,60-0,70 1 (Normliter) pro g oTS (am Lauftag 822d z.B. 0,665) gemessen. Für den Lauftag 822 (Fuzzy-Lauftag 65) ergab sich bei 750 ml Substratzugabe pro 5,7 1 Reaktorinhalt mit 7,0 g oTS/l Silage (OLR = 9,2 g oTS • l⁻¹ • d⁻¹) eine volumetrische Gasproduktion von etwa 6,0 1/1 Reaktorvolumen und eine hydraulische Retentions- oder Verweildauer HRT von 7,6 d.

Das Gas wies einen Methangehalt von etwa 56% auf (Meßgerät mit Druck- und Temperaturkorrektur), wobei sich aufgrund der Stöchiometrie aus Kohlenhydraten max. 50% Methan bilden kann. Die Differenz kommt eventuell aufgrund von Carbonatbildung zustande, die zu einer Aufkonzentrierung von Methan im Abgas führt. Dadurch ergeben sich gleichzeitig entsprechend reduzierte Gasmengen. Die spezifische Gasbildung wird in der Literatur häufig deutlich höher beschrieben. Hierbei handelt es sich aber wahrscheinlich um ungenaue Angaben, die auf unzuverlässigen Messungen beruhen. Bei den beschriebenen Versuchen wurde zur exakten volumetrischen Gasmessung ein Milligascounter®, Hochschule für Angewandte Wissenschaften Hamburg, Lohbrügger Kirschstraße 65, D-21033 Hamburg-Bergedorf, eingesetzt.

Setzt man für die organische Trockensubstanz oTS alleinig Kohlenhydrate mit einem Energiegehalt von 16 kJ/g ein und rechnet bei 1 1 Methan mit 35,9 kJ Energiegehalt, so läßt sich der Abbau leicht überprüfen, da nicht mehr Energie entstehen kann, als zuvor eingesetzt wurde. Mit dem Mittelwert von 0,665 Litern Biogas/g oTS (Tag 822) erhält man mit 56 % Methangehalt und bei 9,2g oTS • 1⁻¹ • d⁻¹ Silagezugabe 123 kJ als Methan/Tag, wobei 9,2 g oTS etwa 147,2 W entsprechen. Unter Berücksichtigung der Tatsache, daß etwa ein Drittel der oTS von Silage als teiloxidierte Essigsäure und Milchsäure vorliegen, deren energetische Werte unterhalb derjenigen von Kohlenhydraten liegen, ist von einem vollständigen Abbau auszugehen. Auch die Konstanthaltung einer vergleichsweise geringen Konzentration an Abbauprodukten im Reaktor (s5 mmol/1)spricht für einen vollständigen Abbau.

Demgegenüber produzierte beispielsweise eine "klassische" zweistufige Betriebsanlage in Nordrhein-Westfalen, Deutschland, aus 450 m³ reiner Silage (ohne Gülle etc.) täglich etwa 500 m³ Biogas, was einer Gasbildungsrate von nur 1,1 l • 1⁻¹ • d⁻¹ entspricht. Mit dem erfindungsgemäßen Verfahren ergaben sich bei den beschriebenen Versuchen jedoch 6,0 l • 1⁻¹ • d⁻¹. Das entspricht einer Steigerung um den Faktor 5,4.

Der oTS Gehalt der Silage der zweistufigen Betriebsanlage betrug zwischen 8,1 und 9,5 % oTS, was einer Beladungsrate von 7,9 - 9,45 m³/ (500 m³ • d) bzw. einer verweildauer HRT von 53 - 63 d, d.h. im Mittel von 58 d, entsprach. Dies ergibt für die Laboranlage, die mit einer Silage von lediglich 7,0 g oTR/l betrieben wurde und dabei 7,6 d HRT erzielte (s. oben) einen weiteren Steigerungsfaktor von 7,6. Insgesamt ergibt sich daher eine Steigerung der Raumzeitausbeute um mehr als das 40fache.

### 2. Thermophile Vergärung

Der oben unter Beispiel 1 beschriebene Versuch wurde unter thermophilen Bedingungen fortgesetzt. Hierzu wurde die Temperatur auf 42 °C eingestellt. Dank der eingesetzten Regelung Fuzzy-Regelung blieb die Vergärung vollkommen stabil (s. Fig. 3), bei nur 7,6 Tagen verweilzeit (HRT). Der Betrieb bei erhöhter Temperatur (ab etwa 40 °C) weist den Vorteil auf, daß unter diesen Bedingungen das Wachstum von Hefen, die den Vergärungsprozeß empfindlich stören können, wirksam unterbunden werden kann, während der gewünschte Vergärungsprozeß durch die methanbildende Bakterienflora bei Temperaturen bis 45 °C nicht beeinträchtigt wird.

Über die Reaktortage 971-1001 (s. Fig. 4) wurde auf die noch kürzere Verweilzeit HRT von 6,5 Tage geschaltet. Die Vergärung blieb auch hier vollkommen stabil (untere HRT-Linie).

Im weiteren Verlauf des Versuches (Reaktortage 1031-1057, nicht dargestellt) wurde eine noch niedrigere verweildauer HRT von 5,43 Tagen eingestellt. Der Gärverlauf blieb immer noch stabil.

Von den 2 Parallelreaktoren, die ebenfalls genauso lange mit Rübenmussilage als Monosubstrat betrieben wurden, lief ein Reaktor lediglich mit einer pH-Sonde und den sonstigen Randbedingungen der Regelung (Dreifachdosage pro Tag, Präfixierung der Verweilzeit und Festlegen der Minimal- und Maximaldosagemenge pro Fütterungszyklus). Die Reaktorergebnisse bzw. Raumzeitausbeuten waren fast genauso hoch und auch so stabil, wie die des Reaktors mit der Regelung über insgesamt drei Messgrößen (pH, spezifische Gasbildungsrate und Methangehalt). Instabilitäten, z.B. durch äußere Faktoren wie technisch bedingte Pannen, konnten jedoch bei einer Regelung mit drei Messgrößen besser aufgefangen werden.

## Patentansprüche

1. Verfahren zur anaeroben Vorgärung von Biomasse als Substrat, insbesondere von pflanzlichen nachwachsenden Rohstoffen oder Speiseresten, bei dem methanhaltiges Biogas gebildet wird, und bei dem einem Reaktor kontinuierlich Substrat zugeführt und kontinuierlich mindestens ein Parameter erfaßt,wird, **dadurch gekennzeichnet, daß** die Regelung eine multivariable Regelung ist, bei der als einzige Eingangsvariablen die folgenden Parameter eingesetzt werden:
a) der pH-Wert im Reakter und
b) die Methankonzentration im gebildeten Biogas und
c) die spezifische Biogasbildungsrate.

2. Verfahren nach Anspruch 1, **dadurch gekennzeichnet, daß** die Regelung eine Fuzzy-Regelung ist.

3. Verfahren nach einem der vorhergehenden Ansprüche, **dadurch gekennzeichnet, daß** das Verfahren im Temperaturbereich von 4-70 °C betrieben wird.

4. Verfahren nach Anspruch 3, **dadurch gekennzeichnet, daß** das Verfahren im Temperaturbereich von 25-40 °C, bevorzugt bei 37 °C betrieben wird.

5. Verfahren nach Anspruch 3, **dadurch gekennzeichnet, daß** das Verfahren im Temperaturbereich von >40-65 °C, bevorzugt >40-50 °C, besonders bevorzugt 42 -45 C betrieben wird.

6. Verfahren nach einem der vorhergehenden Ansprüche, **dadurch gekennzeichnet, daß** das Substrat dem Reaktor in einer Rate zugeführt wird, daß eine durchschnittliche Verweildauer des Substrats in dem Reaktor von ≤ 10 Tagen, bevorzugt ≤ 8 Tagen, besonders bevorzugt ≤ 6 Tagen resultiert (substratabhängig: bezogen auf pflanzliche kohlenhydrathaltige Silagen. Nicht bei stark eiweiss- oder stark fetthaltigen Substraten).

7. Verfahren nach einem der vorhergehenden Ansprüche, **dadurch gekennzeichnet, daß** als resultierende Ausgangsvariable der Regelung die Beladungsrate mit organischem Material (OLR) verwendet wird.

8. Verfahren nach einem der vorhergehenden Ansprüche, **dadurch gekennzeichnet, daß** der pH-Wert im neutralen Bereich, d. h. im Bereich zwischen pH 6 und pH 8, bevorzugt bei einem pH-Wert von etwa 6,8 bis 7,1, gehalten wird.

9. Verfahren nach einem der vorhergehenden Ansprüche, **dadurch gekennzeichnet, daß** das Substrat dem Reaktor mit einer Rate von ≥ 5 g oTS · 1⁻¹ · d⁻¹, bevorzugt von ≥ 10 g oTS · 1⁻¹ · d⁻¹, zugeführt wird.

10. Verfahren nach einem der vorhergehenden Ansprüche zur Vergärung von fester und gelöster Biomasse, wobei die Biomasse ausgewählt ist aus der Gruppe, bestehend aus pflanzlicher Biomasse, hocheiweißhaltigen Substraten, Speiseresten, Schlempen, Faulschlämme, Abwässern und Silage.

11. Verfahren nach einem der vorhergehenden Ansprüche, **dadurch gekennzeichnet, daß** das Substrat ein festes Substrat ist.

12. Verfahren nach einem der vorhergehenden Ansprüche, **dadurch gekennzeichnet, daß** das Substrat dem Reaktor in Intervallen zugeführt wird.

13. Verfahren nach einem der vorhergehenden Ansprüche, **dadurch gekennzeichnet, daß** das Verfahren einstufig betrieben wird.

14. Verfahren nach einem der vorhergehenden Ansprüche, **dadurch gekennzeichnet, daß** die Verweildauer des Substrates im Reaktor substratabhängig gewählt wird.

## Claims

1. Process for the anaerobic fermentation of biomass as substrate, in particular of plant renewable raw materials or leftovers, wherein methane-containing biogas is formed, and wherein a reactor is continuously fed with substrate and continuously at least one parameter is captured, **characterized in that** the control is a multivariable control in which the following parameters are used as sole input variables:
a) the pH-value in the reactor, and
b) the methane concentration in the formed biogas, and
c) the specific biogas formation rate.

2. Process according to claim 1, **characterised in that** the control is a fuzzy control.

3. Process according to one of the preceding claims, **characterised in that** the process is carried out in the temperature range of 4-70 °C.

4. Process according claim 3, **characterised in that** the process is carried out in the temperature range of 25-40 °C, preferably at 37 °C.

5. Process according claim 3, **characterised in that** the process is carried out in the temperature range of >40-65 °C, preferably >40-50 °C, especially preferred 42-45 °C.

6. Process according to one of the preceding claims, **characterised in that** the reactor is fed with the substrate at a rate such that an average retention time of the substrate in the reactor of ≤ 10 days, preferably ≤ 8 days, especially preferred ≤ 6 days, results (depending on the substrate: relating to carbohydrate-containing plant silages. Not with highly protein- or highly fat-containing substrates).

7. Process according to one of the preceding claims, **characterised in that** as resulting output variable of the control the loading rate with organic material (OLR) is used.

8. Process according to one of the preceding claims, **characterised in that** the pH-value is maintained in the neutral range, i.e. in the range between pH 6 and pH 8, preferably at a pH-value of 6.8 to 7.1.

9. Process according to one of the preceding claims, **characterised in that** the reactor is fed with the substrate at a rate of ≥ 5 g oDS · 1⁻¹ · d⁻¹, preferably of ≥ 10 g oDS · 1⁻¹ · d⁻¹.

10. Process according to one of the preceding claims for the fermentation of solid and dissolved biomass, wherein the biomass is selected from the group consisting of plant biomass, high-protein-containing substrates, leftovers, distiller's wash, digested sludges, wastewaters and silage.

11. Process according to one of the preceding claims, **characterised in that** the substrate is a solid substrate.

12. Process according to one of the preceding claims, **characterised in that** the reactor is fed with the substrate in intervals.

13. Process according to one of the preceding claims, **characterised in that** the process is operated in a single stage.

14. Process according to one of the preceding claims, **characterised in that** the retention time of the substrate in the reactor is chosen depending on the substrate.

## Revendications

1. Procédé de fermentation anaérobie d'un substrat constitué de biomasse, notamment de matières premières végétales renouvelables ou de restes de nourriture, ledit procédé permettant de produire du biogaz à teneur en méthane et consistant à introduire ledit substrat de manière continue dans un réacteur tout en enregistrant de manière continue au moins un paramètre, **caractérisé en ce que** l'on met en place une régulation de type régulation à variables multiples dans laquelle les paramètres suivants constituent les seules variables d'entrée:
a) le pH au sein du réacteur, et
b) la concentration de méthane dans le biogaz produit, et
c) le taux spécifique de production de biogaz.

2. Procédé selon la revendication 1, **caractérisé en ce que** ladite régulation est une régulation à logique floue.

3. Procédé selon l'une des revendications précédentes, **caractérisé en ce que** ledit procédé en mis en oeuvre à une température comprise entre 4 et 70 °C.

4. Procédé selon la revendication 3, **caractérisé en ce que** ledit procédé en mis en oeuvre à une température comprise entre 25 et 40 °C, de préférence égale à 37 °C.

5. Procédé selon la revendication 3, **caractérisé en ce que** ledit procédé en mis en oeuvre à une température comprise entre > 40 et 65 °C, de préférence entre > 40 et 50 °C, avec une préférence particulière entre 42 et 45 °C.

6. Procédé selon l'une des revendications précédentes, **caractérisé en ce que** ledit substrat est introduit dans le réacteur avec un débit tel qu'au sein du réacteur, ledit substrat présente une durée de séjour moyenne de ≤ 10 jours, préférentiellement de ≤ 8 jours, avec une préférence particulière de ≤ 6 jours (en fonction du substrat: les indication se rapportent à des ensilages végétaux contenant des glucides. Les substrats à haute teneur en protéines ou lipides ne sont pas visés).

7. Procédé selon l'une des revendications précédentes, **caractérisé en ce que** le taux d'alimentation en matière organique (OLR) est utilisé comme variable de sortie qui résulte de ladite régulation.

8. Procédé selon l'une des revendications précédentes, **caractérisé en ce que** l'on maintient le pH dans une gamme neutre, c'est-à-dire comprise entre pH 6 et pH 8, le pH étant préférentiellement maintenu entre 6,8 et 7,1.

9. Procédé selon l'une des revendications précédentes, **caractérisé en ce que** ledit substrat est introduit dans le réacteur avec un débit ≥ 5 g oMS · 1⁻¹ · d⁻¹, de préférence ≥ 10 g oMS · 1⁻¹ · d⁻¹.

10. Procédé selon l'une des revendications précédentes, permettant de fermenter de la biomasse solide ou mise en solution, ladite biomasse étant choisie dans le groupe constitué de biomasse végétale, de substrats à haute teneur en protéines, de restes de nourriture, de drêches, de boues d'épuration stabilisées par fermentation anaérobie, d'eaux usées et d'ensilage.

11. Procédé selon l'une des revendications précédentes, **caractérisé en ce que** ledit substrat est un substrat solide.

12. Procédé selon l'une des revendications précédentes, **caractérisé en ce que** ledit substrat est introduit dans le réacteur selon un régime d'intervalles.

13. Procédé selon l'une des revendications précédentes, **caractérisé en ce que** ledit procédé en mis en oeuvre en une seule étape.

14. Procédé selon l'une des revendications précédentes, **caractérisé en ce que** la durée de séjour du substrat dans le réacteur est choisie en fonction du substrat.
